# NEUE EUROPÄISCHE PATENTSCHRIFT

(11) **EP 0 532 557 B2**
(45) Veröffentlichungstag und Bekanntmachung des Hinweises auf die Entscheidung über den Einspruch: **15.07.1998**
(45) Hinweis auf die Patenterteilung: 14.12.1994
(21) Anmeldenummer: 91910096.6
(22) Anmeldetag: 27.05.1991
(51) Int. Cl.: C07C 305/10, C07C 303/24, C11D 1/16

(54) **VERFAHREN ZUR HERSTELLUNG VON GLYCERINETHERSULFATEN**
PROCESS FOR PRODUCING GLYCERIN ETHER SULPHATES
PROCEDE DE PRODUCTION DE SULFATES D'ETHERS GLYCERIQUES

(30) Priorität: 06.06.1990 US 534592
(43) Veröffentlichungstag der Anmeldung: 24.03.1993
(73) Patentinhaber: Henkel Kommanditgesellschaft auf Aktien, 40191 Düsseldorf (DE)
(72) Erfinder: FABRY, Bernd, D-4052 Korschenbroich (DE); GRUBER, Bert, D-5012 Bedburg (DE); TUCKER, Fames, Robinson, Laguna Hills, CA 92656 (US); GIESEN, Brigitte, D-4000 Düsseldorf (DE)
(86) Internationale Anmeldenummer: EP9100974
(87) Internationale Veröffentlichungsnummer: WO9118871

(56) Entgegenhaltungen:
- EP-A- 0 011 311
- CH-A- 205 160
- DE-C- 757 749
- US-A- 4 217 296
- JAOCS January 1978, vol. 51, pp. 128-133, Lanteri
- Kirk-Othmer Encyclopedia of Chemical Technology, 1983, 3rd ed., vol. 22, pp. 1-38

## Beschreibung

Gegenstand der Erfindung ist ein Verfahren zur Herstellung von Glycerinethersulfaten durch Sulfatierung von Glycerinethern mit gasförmigem Schwefeltrioxid.

### Stand der Technik

Aus der deutschen Patentschrift **DE-PS 757749** sowie der japanischen Patentschrift **JP 82/44673** ist bekannt, daß Glycerinethersulfate als Hilfsmittel in der Textil- und Ledertechnik sowie zur Herstellung von Farbstoffen Verwendung finden können. In der genannten deutschen Patentschrift wird offenbart, daß man 1,3-Glycerindialkylether mit Hilfe von Schwefelsäure, Chlorsulfonsäure, Amidosulfonsäure, Addukten von Pyridin an Schwefeltrioxid oder gasförmigem Schwefeltrioxid in einem inerten Lösungsmittel, wie beispielsweise Dimethylformamid, Pyridin oder Dichlorethan, in die sekundären Sulfate überführen kann. Die genannten Verfahren sind jedoch mit technischen Nachteilen verbunden. So führt die Verwendung von Schwefelsäure als Sulfiermittel zu einer unerwünscht hohen Elektrolytbelastung der Produkte. Die Sulfatierung mittels Chlorsulfonsäure führt zwangsläufig zur Bildung hochkorrosiver Salzsäure, während die Verwendung von Amidosulfonsäure nur die Herstellung von Ammoniumsalzen zuläßt. Addukte von Pyridin an Schwefeltrioxid sind schwer zu handhaben und kommen für eine technische Realisierung nicht in Frage. Bei Einsatz von gasförmigem Schwefeltrioxid in einem inerten Lösungsmittel besteht schließlich das Problem, das Lösungsmittel mit hohem technischen Aufwand vom Produkt abzutrennen zu müssen. Aus der Patentschrift **US 4,217,296** ist ein Verfahren zur Herstellung von sulfatierten Glycerinethern unter Einsatz von Schwefeltrioxid als Sulfatierungsmittel bekannt. Gemäß der Lehre dieser Schrift ist es zwingend, die Einsatzstoffe vor dem Inkontaktbringen mit dem gasförmigem SO₃ in einem inerten Lösungsmittel vorzulegen und auf diese Weise lokale Überhitzungen und die Bildung von Zersetzungsprodukten zu vermeiden.

Der Erfindung lag daher die Aufgabe zugrunde, ein Verfahren zur Herstellung von Glycerinethersulfaten zu entwickeln, das frei von den geschilderten Nachteilen ist.

### Beschreibung der Erfindung

Gegenstand der Erfindung ist ein Verfahren zur Herstellung von Glycerinethersulfaten durch Umsetzung von Glycerinethern mit Sulfiermitteln, welches sich dadurch auszeichnet, daß man eine flüssige Phase, ausschließlich bestehend aus Glycerinethern der Formel **(I)**, 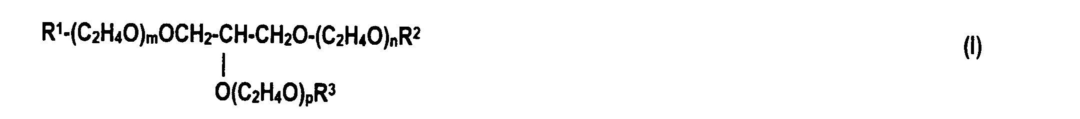 in der R¹ für einen Alkylrest mit 4 bis 22 Kohlenstoffatomen steht, R² und R³ Wasserstoff oder einen Alkylrest mit 4 bis 22 Kohlenstoffatomen darstellen, mit der Bedingung, daß mindestens einer der beiden Reste für Wasserstoff sowie m, n und p unabhängig voneinander für 0 oder eine ganze Zahl von 1 bis 20 steht und die Summe von m, n und p 0 ist oder eine Zahl von 1 bis 20 bedeutet, bei Temperaturen im Bereich von 35 bis 90°C mit gasförmigem Schwefeltrioxid sulfatiert und die sauren Sulfatierungsprodukte anschließend mit wäßrigen Basen neutralisiert.

Die Erfindung schließt die Erkenntnis mit ein, daß die wäßrigen Lösungen der Glycerinethersulfate, die nach dem erfindungsgemäßen Verfahren erhalten werden, klar und daher mit Vorteil einzusetzen sind, während Lösungen von Glycerinethersulfaten, die nach den Verfahren des Stands der Technik hergestellt werden, trübe sind.

### Glycerinether

Glycerinether der Formel **(I)** stellen bekannte Verbindungen dar und lassen sich nach den gängigen Methoden der präparativen organischen Chemie herstellen. Geeignete Verfahren zur Herstellung von Glycerinethern stellen beispielsweise die Umsetzungen von Glycerin oder Anlagerungsprodukten von durchschnittlich 1 bis 20 Mol Ethylenoxid an Glycerin mit Alkylhalogeniden **[DE-A1 2800710]**, Olefinepoxiden oder Butadien und Wasserstoff in Gegenwart von Übergangsmetallkatalysatoren **[J.Mol. Catal., 10, 247 (1981), Organometallics 5, 473, 514 (1986)]** dar.

### Glycerinethersulfate

Glycerinethersulfate mit besonders günstigen anwendungstechnischen Eigenschaften werden erhalten, wenn R¹ in Formel **(I)** für einen Alkylrest mit 8 Kohlenstoffatomen und R² entweder gleich R¹ ist oder für Wasserstoff steht. Glycerinethersulfate, die sich durch ein besonderes vorteilhaftes Wasch- und Spülvermögen auszeichnen, leiten sich von Glycerinethern der Formel **(I)** ab, in der m, n und p für 0 stehen. Glycerinethersulfate, die sich durch ein besonders vorteilhaftes Schaumvermogen und hohe Wasserlöslichkeit auszeichnen, leiten sich von Glycerinethern der Formel **(I)** ab, in der m, n und p für eine Zahl von 2 bis 5 und die Summe aus m, n und p für einen Zahl von 2 bis 10 stehen.

### Sulfatierung, Neutralisation und Bleiche

Die Umsetzung der Glycerinether mit gasförmigem Schwefeltrioxid kann in der für Fettsäureniedrigalkylester bekannten Weise **[J.Falbe (ed.), "Surfactants in consumer products"; Springer Verlag, Berlin-Heidelberg, 1987, S.61]** erfolgen, wobei Reaktoren, die nach dem Fallfilmprinzip arbeiten, bevorzugt sind. Dabei wird das Schwefeltrioxid mit einem inerten Gas, vorzugsweise Luft oder Stickstoff verdünnt und in Form eines Gasgemisches, welches das Sulfieragens in einer Konzentration von 1 bis 8, insbesondere 2 bis 5 Vol.-% enthält, eingesetzt. Das molare Einsatzverhältnis von Glycerinether zu Schwefeltrioxid beträgt 1 : 0,95 bis 1 : 1,8, vorzugsweise jedoch 1 : 1,0 bis 1 : 1,5. und insbesondere 1 : 1,3 bis 1 : 1,5. Die Sulfierreaktion wird bei Temperaturen von 35 bis 90, vorzugsweise 35 bis 80°C durchgeführt.

Die bei der Sulfatierung anfallenden sauren Sulfierprodukte werden in wäßrige Basen eingerührt, neutralisiert und auf einen pH-Wert von 6,5 bis 8,5 eingestellt. Die Neutralisation wird mit Basen ausgewählt aus der von Alkalimetallhydroxiden wie Natrium-, Kalium- und Lithiumhydroxid, Erdalkalimetalloxiden und -hydroxiden wie Magnesiumoxid, Magnesiumhydroxid, Calciumoxid und Calciumhydroxid, Ammoniak, Mono-, Di- und Tri-C₂₋₄-Alkanolaminen, beispielsweise Mono-, Di- und Triethanolamin sowie primären, sekundären oder tertiären C₁₋₄-Alkylaminen gebildeten Gruppe durchgeführt. Die Neutralisationsbasen gelangen dabei vorzugsweise in Form 5 bis 55 Gew.-%iger wäßriger Lösungen zum Einsatz, wobei 25 bis 50 gew.-%ige wäßrige Natriumhydroxidlösung bevorzugt ist.

Die nach dem erfindungsgemäßen Verfahren erhältlichen Glycerinethersulfate liegen nach Neutralisation als wäßrige Lösungen mit einem Aktivsubstanzgehalt von 20 bis 80, vorzugsweise 30 bis 50 Gew.-% vor. Bei Umsetzung von Glycerinmonoalkylethern mit Schwefeltrioxid werden Gemische der korrespondierenden primären und sekundären Monosulfate erhalten. In der Reaktionsmischung können ferner Disulfate und cyclische Schwefelsäureester enthalten sein. Die Produkte sind ohne Bleiche hellfarbig, blank und niedrigviskos. Setzt man Glycerindialkylether in die Sulfatierung ein, werden sekundäre Monosulfate erhalten, die auch ohne Bleiche als hellfarbige, opake, viskose Pasten vorliegen.

Die Sulfatierungsprodukte können nach der Neutralisation in an sich bekannter Weise durch Zusatz von Wasserstoffperoxid- oder Natriumhypochloritlösung gebleicht werden, um eine für viele Anwendungen erwünschte weitere Farbaufhellung zu erreichen. Dabei werden, bezogen auf den Feststoffgehalt in der Lösung der Sulfierprodukte, 0,2 bis 2 Gew.-% Wasserstoffperoxid, berechnet als 100 Gew.-%ige Substanz, oder entsprechende Mengen Natriumhypochlorit eingesetzt. Der pH-Wert der Lösungen kann unter Verwendung geeigneter Puffermittel, z. B. mit Natriumphosphat oder Citronensäure konstant gehalten werden. Zur Stabilisierung gegen Bakterienbefall empfiehlt sich ferner eine Konservierung, z. B. mit Formaldehydlösung, p-Hydroxybenzoat, Sorbinsäure oder anderen bekannten Konservierungsstoffen.

### Gewerbliche Anwendbarkeit

Die nach dem erfindungsgemäßen Verfahren erhältlichen Glycerinethersulfate zeigen hervorragende Detergenseigenschaften und eignen sich zur Herstellung oberflächenaktiver Mittel, insbesondere manueller Geschirrspülmittel und flüssiger sowie pulverförmiger Waschmittel.

### Beispiele

### Herstellung der Ausgangsstoffe:

**A) 1-n-Octyl-glycerinether.** In einem 400 ml Glasautoklaven mit Magnetrührung wurden 0.0038 g (0.0125 mmol) Palladium(ll)actylacetonat, 0,0066 g (0.025 mmol) Triphenylphosphin, 28,8 g (313 mmol) Glycerin und 35 ml 2-Propanol vorgelegt und insgesamt dreimal evakuiert und mit Argon beaufschlagt. Im Anschluß daran wurden mit Hilfe eines Hebers 37,8 g (700 mmol) Butadien-1,3 in den Autoklaven überführt. Der Autoklav wurde verschlossen und 12 h bei 70°C gehalten. Nach Abkühlen und Entspannen wurde eine farblose Flüssigkeit erhalten, die in einen 400 ml Stahlautoklaven mit Magnetrührung überführt wurde. Das ungesättigte Reaktionsgemisch wurde mit 0,5 Gew.-% - bezogen auf die Gesamtmenge des Ausgangsmaterials - Raney-Nickel versetzt und über einen Zeitraum von 2 h bei 180°C hydriert. Nach Abkühlen und Entspannen wurde der Katalysator abgetrennt und eine farblose Flüssigkeit der folgenden Zusammensetzung erhalten:

| | |
|---|---|
| 1-n-Octylglycerinether | 34,0 Gew.-% |
| 1,3- und 1,2-Di-n-octylglycerinether | 21,7 Gew.-% |
| 1,2,3-Tri-n-octylglycerinether | 2,1 Gew.-% |
| Glycerin | 11,3 Gew.-% |
| Octen | 2,8 Gew.-% |
| 2-Propanol | 28,1 Gew.-% |

Das Produkt wurde ohne weitere Reinigung in die Sulfatierung eingesetzt.
B) **1,3- bzw. 2,3-Di-n-octylglycerinether**. A) wurde unter Einsatz von 10,3 g (112 mmol) Glycerin, und 13,5 g (250 mmol) Butadien-1,3 wiederholt. Anstelle des Propanol-2 wurde 1,0 g (13 mmol) Propandiol-1,2 eingesetzt. Das Reaktionsprodukt wurde nach der Hydrierung als leicht gelb gefärbte Flüssigkeit der folgenden Zusammensetzung erhalten.

| | |
|---|---|
| 1,3- und 1,2-Di-n-octylglycerinether | 43,0 Gew.-% |
| 1-n-Octylglycerinether | 15,0 Gew.-% |
| 1,2,3-Tri-n-octylglycerinether | 17,8 Gew.-% |
| Propandiol-1,2 | 8,3 Gew.-% |
| 1-n-Octyl-propandiol-1,2-ether | 5,1 Gew.-% |
| 1,3-Di-n-octylpropandiol-1,2-ether | 0,8 Gew.-% |
| Octen | 10,0 Gew.-% |

**C) C**_{**12/14**}**-Alkylglycerin+2EO-ether.** In einem 1-l-Dreihalskolben mit Rührer, Innenthermometer und Rückflußkühler wurden 180 g (1 Mol) eines Anlagerungsproduktes von durchschnittlich 2 Mol Ethylenoxid an Glycerin vorgelegt und mit 218 g (2 Mol) C_{12/14}-Alkylchlorid und 112 g Kaliumhydroxid-Schuppen versetzt. Anschließend wurde die Reaktionsmischung unter Rühren 5 h bei 110°C gehalten. Nach dem Abkühlen wurde die basische Reaktionsmischung mit Salzsäure neutralisiert und das überschüssige Alkylchlorid abdestilliert. Die Ausbeute an Glycerinether betrug 95 % bezogen auf die theoretische Menge.

### Beispiele 1 bis 4:

**Sulfatierung von Glycerinethern**. In einem 1-l-Sulfierreaktor mit Mantelkühlung und Gaseinleitungsrohr wurden 1 mol Glycerinether A, B oder C bei T = 35 bzw. 80°C mit 0,95 bis 1,2 mol Schwefeltrioxid umgesetzt. Das Schwefeltrioxid wurde durch Erhitzen aus einer entsprechenden Menge 65 Gew.-%igen Oleums ausgetrieben, mit Stickstoff auf eine Konzentration von 5 Vol.-% verdünnt und innerhalb von 50 min in das Ausgangsprodukt eingeleitet. Das rohe Sulfierprodukt wurde anschließend mit wäßriger 25 Gew.-%iger Natronlauge neutralisiert. Die Reaktionsbedingungen sowie die Kenndaten der Produkte sind in Tabelle 1 zusammengefaßt.

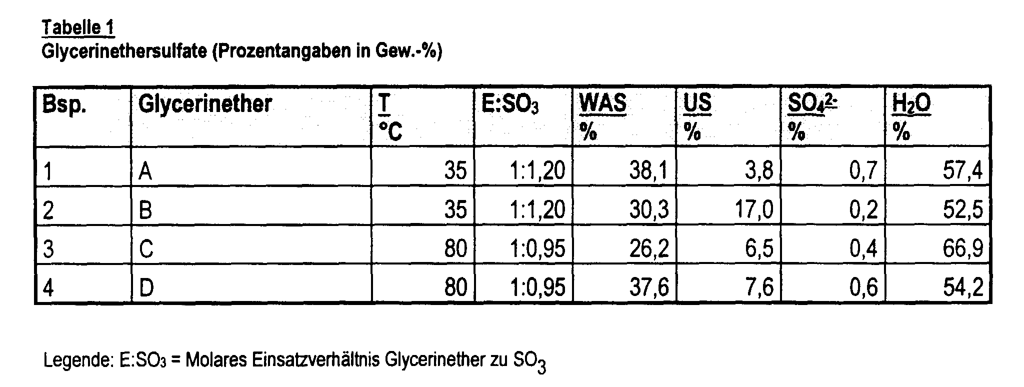

Der Aniontensidgehalt (WAS) und die unsulfierten Anteile (US) wurden nach den DGF-Einheitsmethoden, Stuttgart, 1950-1984, H-III-10 bzw. G-ll-6b ermittelt. Der Sulfatgehalt wurde als Natriumsulfat berechnet, die Bestimmung des Wassergehaltes erfolgte nach der Fischer-Methode.

### Anwendungstechnische Beispiele:

**Reinigungsvermögen.** Zur Demonstration der Reinigungsleistung wurde der sogenannte Tellertest durchgeführt **[Fette,Seifen,Anstrichm., 74, 163 (1972)]**. Es wurde mit Wasser von 50°C bei 3°d bzw. 16°d und einem Einsatz von 0,15 g Glycerinethersulfat pro Liter Wasser sowie einer Rindertalganschmutzung gearbeitet. Die Talganschmutzung wurde mit 2 g/Teller (Untertasse von ca. 14 cm Durchmesser, verteilt in der Tassenvertiefung) eingesetzt; die angeschmutzten Teller wurden nach 24 h Lagerung bei Raumtemperatur unter den angegebenen Bedingungen gespült. Die Ergebnisse sind in Tabelle 2 zusammengefaßt:

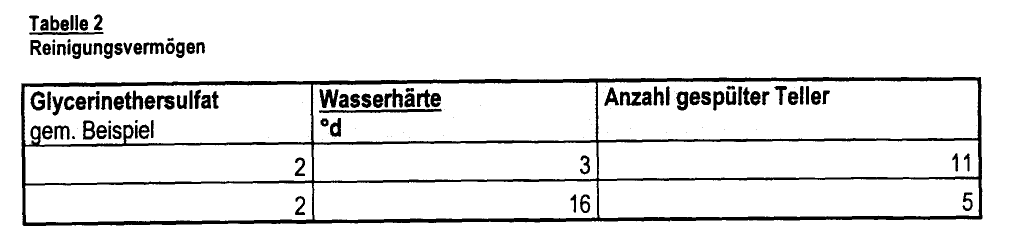

**Waschvermögen**. Die Untersuchung des Waschvermögens wurde in einem Launderometer an einer Staub-Hautfett-Anschmutzung auf Polyester- Baumwoll(veredelt)-Gewebe durchgeführt. Die Aufhellung des gewaschenen Gewebes erfolgte durch photometrische Remissionsmessung mit einem Elrepho RFC-3/24-Gerät gegenüber einem Bariumsulfat- Standard, dessen Remission zu 100 % gesetzt wurde. Die Angabe erfolgt in %-Remission (%-R). Nachfolgend sind die Rezepturen für ein phosphaffreies Pulverwaschmittel (Rezeptur A) und ein Flüssigwaschmittel (Rezeptur B) sowie die Waschbedingungen aufgeführt. Die Ergebnisse der Waschversuche sind in Tabelle 3 zusammengefaßt.

### Rezeptur A:

| | |
|---|---|
| 8,0 Gew.-% | Glycerinethersulfat-Na-Salz, gemäß Bsp.1 bzw. 2 |
| 4,0 Gew.-% | Dehydol® LT7 (Henkel, C_{12/18}-Fettalkohol+7EO-Addukt) |
| 1,5 Gew.-% | Talgseife, gehärtet |
| 24,0 Gew.-% | Sasil® Zeolith A (Henkel) |
| 10,0 Gew.-% | Soda |
| 3,0 Gew.-% | Wasserglas |
| 15,0 Gew.-% | Natriumperborat |
| 20,0 Gew.-% | Natriumsulfat |

### Rezeptur B:

| | |
|---|---|
| 13,0 Gew.-% | Glycerinethersulfat-Na-Salz, gemäß Beispiel 1 bzw. 2 |
| 15,0 Gew.-% | Dehydol® LT7 |
| 13,0 Gew.-% | Kokosseife |
| 5,0 Gew.-% | Triethanolamin |
| 0,5 Gew.-% | Citronensäure |

Waschbedingungen: Flotte : 250 ml; Flottenbelastung : 1 Gew.-Teil Gewebe : 30 Gew.-% Teile Wasser; Dosierung : 10 g/l; Wasserhärte : 16°d; Bestimmung : Mittel von 12 Bestimmungen

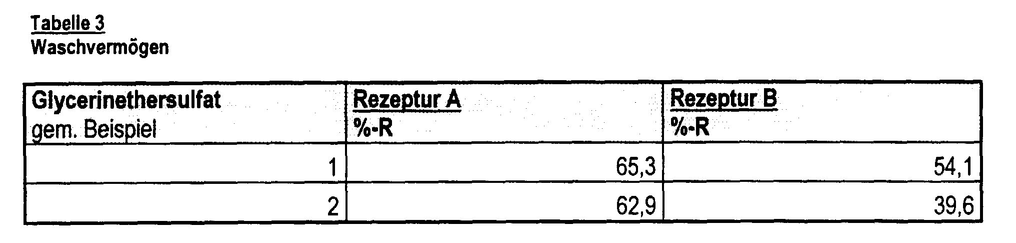

## Patentansprüche

1. Verfahren zur Herstellung von Glycerinethersulfaten durch Umsetzung von Glycerinethern mit Sulfiermitteln, **dadurch gekennzeichnet**, daß man eine flüssige Phase, ausschließlich bestehend aus Glycerinethern der Formel **(I)**, 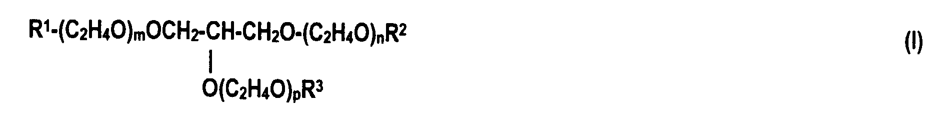 in der R¹ für einen Alkylrest mit 4 bis 22 Kohlenstoffatomen steht, R² und R³ Wasserstoff oder einen Alkylrest mit 4 bis 22 Kohlenstoffatomen darstellen, mit der Bedingung, daß mindestens einer der beiden Reste für Wasserstoff sowie m, n und p unabhängig voneinander für 0 oder eine ganze Zahl von 1 bis 20 steht und die Summe von m, n und p 0 ist oder eine Zahl von 1 bis 20 bedeutet, bei Temperaturen im Bereich von 35 bis 90°C mit gasförmigem Schwefeltrioxid sulfatiert und die sauren Sulfatierungsprodukte anschließend mit wäßrigen Basen neutralisiert.

2. Verfahren nach Anspruch 1, **dadurch gekennzeichnet**, daß man Glycerinether der Formel **(I)** einsetzt, in der R¹ für einen Alkylrest mit 8 Kohlenstoffatomen steht.

3. Verfahren nach den Ansprüchen 1 und 2, **dadurch gekennzeichnet**, daß man Glycerinether der Formel **(I)** einsetzt, bei denen m, n und p für 0 stehen.

4. Verfahren nach den Ansprüchen 1 und 2, **dadurch gekennzeichnet**, daß man Glycerinether der Formel **(I)** einsetzt, bei denen die Summe (m+n+p) für Zahlen von 2 bis 10 steht.

5. Verfahren nach den Ansprüchen 1 bis 4, **dadurch gekennzeichnet**, daß man die Glycerinether und das Schwefeltrioxid im molaren Verhältnis von 1 : 0,95 bis 1 : 1,8 einsetzt.

## Claims

1. A process for the production of glycerol ether sulfates, characterized in that a liquid phase essentially consisting of glycerol ethers corresponding to formula **(I)** 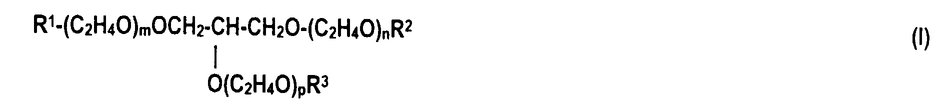 in which R¹ is an alkyl radical containing 4 to 22 carbon atoms, R² and R³ represent hydrogen or an alkyl radical containing 4 to 22 carbon atoms, with the proviso that at least one of the two substituents is hydrogen, and m, n and p independently of one another are 0 or numbers of 1 to 20 and the sum of m, n and p is 0 or a number of 1 to 20, is sulfated at temperatures of from 35 to 90°C with gaseous sulfur trioxide and subsequently neutralized with aqueous bases.

2. A process as claimed in claim 1, **characterized in that** glycerol ethers of formula **(I)**, in which R¹ represents an alkyl radical containing 8 carbon atoms, are used.

3. A process as claimed in claim 1 and 2, **characterized in that** glycerol ethers corresponding to formula (I), in which m, n and p are 0, are used.

4. A process as claimed in claim 1 and 2, **characterized in that** glycerol ethers of formula **(I)**, in which the sum (m+n+p) is a number of 2 to 10, are used.

5. A process as claimed in at least one of claims 1 to 4, **characterized in that** the glycerol ethers and the sulfating agent are used in a molar ratio of 1:0.95 to 1:1.8.

## Revendications

1. Procédé d'obtention de sulfates d'éther de glycéryle caractérisé en ce que l'on sulfate une phase liquide qui contenante seulement un éther de glycéryle de formule **(I)** : 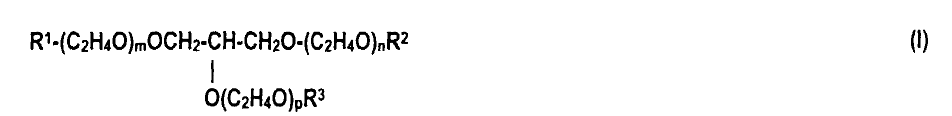 dans laquelle: R¹ représente un radical alcoyle ayant de 4 à 22 atomes de carbone, R² et R³ représentent de l'hydrogène ou un radical alcoyle ayant de 4 à 22 atomes de carbone avec la condition qu'au moins un de deux radicaux repésente die l'hydrogène, ainsi que m, n et p indépendamment l'un de l'autre représentent 0 ou un nombre allant de 1 à 20 et la somme de m, n et p est 0 ou représente un nombre allant de 1 à 20, avec de l'anhydride sulfurique gazeux a une temperature de 35 à 90°C et ensuite on neutralise avec de bases aqueuses.

2. Procédé selon la revendicacion 1, caractérisé en ce que l'on met on oeuvre des éthers de glycéryle de formule **(I)** dans laquelle R¹ représente un radical alcoyle ayant 8 de atomes de carbone.

3. Procède selon des revendicacion 1 et 2, caractérisé en ce que l'on met on oeuvre des éthers de glycéryle de formule **(I)** pour lesquels m, n et p représentent 0.

4. Procéde selon des revendicacion 1 et 2, caractérisé en ce que l'on met on oeuvre des éthers de glycéryle de formule **(I)** pour lesquels la somme (m+n+p) représente un nombre allant de 2 à 10.

5. Procéde selon au moins une des revendications 1 à 4, caractérisé en ce que l'on met on oeuvre l'éther de glycéryle et l'agent de sulfatation dans une rapport molaire allant de 1 : 0,95 à 1 : 1,8.
